# EUROPÄISCHE PATENTSCHRIFT

(11) **EP 1 112 034 B1**
(45) Veröffentlichungstag und Bekanntmachung des Hinweises auf die Patenterteilung: **28.04.2004**
(21) Anmeldenummer: 99942860.0
(22) Anmeldetag: 12.08.1999
(51) Int. Cl.: A61B 19/00

(54) **KOPFKLEMME**
HEAD CLAMPING DEVICE
DISPOSITIF DE SERRAGE POUR UNE TETE

(30) Priorität: 09.09.1998 DE 19841250
(43) Veröffentlichungstag der Anmeldung: 04.07.2001
(73) Patentinhaber: AESCULAP AG & Co. KG, 78532 Tuttlingen (DE)
(72) Erfinder: REINHARDT, Hans, F., CH-4056 Basel (CH); FISCHER, Manfred, D-78532 Tuttlingen (DE); NESPER, Markus, D-78532 Tuttlingen (DE)
(74) Vertreter: Böhme, Ulrich, Dr. Dipl.-Phys.
(86) Internationale Anmeldenummer: PCT/EP1999/005922
(87) Internationale Veröffentlichungsnummer: WO 2000/013604

(56) Entgegenhaltungen:
- DE-C- 4 342 971
- US-A- 2 966 383
- US-A- 4 108 426
- US-A- 5 318 509

## Beschreibung

Die Erfindung betrifft eine Kopfklemme zum Lagern eines Kopfes mit einem aus zwei Rahmenelementen gebildeten Rahmen, die jeweils erste Enden, die beweglich miteinander verbunden sind, und zweite Enden, die Dorne zur Fixierung des Kopfes tragen, aufweisen, wobei die Rahmenelemente relativ zueinander um eine gemeinsame Rotationsachse schwenkbar gelagert sind und der Rahmen eine Schwenkpositionsfixierungsvorrichtung mit mindestens einem Fixierglied an einem der beiden Rahmenelemente umfaßt.

Bei den bereits bekannten Kopfklemmen der eingangs beschriebenen Art wird der Kopf zwischen den beiden Rahmenelementen eingespannt, und zwar durch eine lineare Bewegung der beiden Rahmenelemente relativ zueinander (US-A-5,318,509). Zum Lagern des Kopfes wird dieser zunächst zwischen die beiden Rahmenelemente gebracht, und anschließend werden die beiden Rahmenelemente über jeweils einen Schenkelarm des Rahmenelements axial gegeneinander verschoben. Zu einer Vorfixierung werden die Rahmenelemente relativ zueinander verriegelt. Endgültig eingespannt wird der Kopf mittels einer Feinjustierung der Dorne, bei der diese noch weiter in Richtung auf den Kopf hin bewegt werden.

Die Nachteile dieser Kopfklemmen sind vor allem in dem großen mechanischen Spiel der Rahmenelemente relativ zueinander zu sehen, das um so größer ist, je länger die miteinander in Kontakt stehenden Schenkelarme sind. Darüber hinaus ist die Verstellung und das Nachspannen der Dorne durch Schrauben aufwendig und umständlich. Nachteilig ist auch die erhöhte Bruchgefahr bei vermehrter Belastung, insbesondere durch das Anbringen von Zusatzgeräten. Außerdem sind diese Kopfklemmen für zusätzliche neuroradiologische Untersuchungen untauglich, da sie den Strahlengang erheblich einschränken.

Bei einer weiteren bekannten Kopfklemme sind die beiden Rahmenelemente schwenkbar miteinander verbunden und werden über eine Schwenkpositionsfixierungsvorrichtung relativ zueinander fixiert, die eine Spindel an einem Rahmenelement und ein diese Spindel aufnehmendes Gewinde am anderen Rahmenelement umfaßt (US-A-2,966,383). Es ergibt sich eine sperrige Anordnung, bei der die Schwenkposition der beiden Rahmenelemente nur langsam verändert werden kann, es ist dazu notwendig, die Spindel mehrfach zu verdrehen. Aufgrund der großen Hebelarme ergeben sich außerdem unter Umständen sehr große Kräfte, die auf die Spindelanordnung wirken, so daß die Stabilität dieser Anordnung nicht immer gewährleistet ist.

Es ist Aufgabe der Erfindung, eine Kopfklemme der gattungsgemäßen Art so auszubilden, daß sie schnell verstellbar ist und eine höhere mechanische Stabilität bietet.

Diese Aufgabe wird bei einer Kopfklemme der eingangs beschriebenen Art erfindungsgemäß dadurch gelöst, daß die Schwenkpositionsfixierungsvorrichtung an dem Fixierglied einen Vorsprung aufweist und daß am anderen Rahmenelement korrespondierende Ausnehmungen angeordnet sind, in die der Vorsprung in einer Fixierstellung eingreift und die Rahmenelemente relativ zueinander in ihrer Lage fixiert, und daß das Fixierglied relativ zu den Ausnehmungen von der Fixierstellung in eine Lösestellung bringbar ist, in der die Rahmenelemente relativ zueinander verschwenkbar sind.

Zum Einspannen des Kopfes werden die beiden Rahmenelemente also auch relativ zueinander verschwenkt, und zwar um eine gemeinsame Rotationsachse. Dies ermöglicht es, die Rahmenelemente so klein wie möglich zu dimensionieren, da die Schenkelarme der Rahmenelemente im Gegensatz zu denen von in axialer Richtung verstellbaren Kopfklemmen nicht länger ausgebildet werden müssen, um ein Öffnen der Kopfklemme zu ermöglichen. Durch das relative Verschwenken der Rahmenelemente ist folglich bei geringster Baugröße ein maximaler Öffnungswinkel einstellbar. Dies erleichtert die Handhabung und das Anlegen der Kopfklemme. Ferner wird das Gewicht der Kopfklemme drastisch reduziert, da die Rahmenelemente wesentlich kleiner und kürzer gestaltet werden können. Durch eine so erzielte kleinere Bauform der Kopfklemme wird deren mechanische Stabilität im Vergleich zu den bereits bekannten wesentlich erhöht.

Durch die Ausgestaltung der Schwenkpositionsfixierungsvorrichtung mit einem Vorsprung, der in eine einer Vielzahl von korrespondierenden Ausnehmungen eingreift, ist es möglich, die Rahmenelemente relativ zueinander in einer festgelegten Position zu fixieren. Es ist also allein durch das Zusammenwirken des Fixierglieds mit den Ausnehmungen möglich, die relative Verschwenkung der Rahmenelemente festzulegen, beispielsweise allein durch das Bewegen des Vorsprungs des Fixierglieds in die Ausnehmung. Sobald dann das Fixierglied aus den Ausnehmungen zurückgezogen wird, können die Rahmenelemente wieder beliebig verschwenkt werden.

Bei Kopfklemmen ist es an sich bereits bekannt, Drehverbindungen durch ratschenähnliche Mechanismen zu fixieren (US-A-5,318,509), diese Drehfixierungen sind jedoch nicht zwischen den Rahmenelementen vorgesehen, sondern dienen lediglich der Drehfestlegung der am Kopf anzulegenden Dorne, so daß dieser Druckschrift keine Anregung dafür zu entnehmen ist, die Winkellage der relativ zueinander verschwenkbaren Rahmenelemente durch eine Schwenkpositionsfixierungsvorrichtung festzulegen, die einen Vorsprung umfaßt, der in einen von mehreren korrespondierenden Ausnehmungen eingreift.

Es ist günstig, wenn die Ausnehmungen drehfest mit dem anderen Rahmenelement verbunden sind. Die drehfeste Verbindung hat in erster Linie den Vorteil, die Positionsfixierung möglichst spielfrei zu gestalten, denn ein relativer Freiheitsgrad für eine Bewegung zwischen den Ausnehmungen und dem Rahmenelement der Kopfklemme wird so eliminiert. Außerdem kann auf einen weiteren aufwendigen Feststellmechanismus verzichtet werden, da die Vorsprünge der Fixierglieder des einen Rahmenelements direkt in die Ausnehmungen eingreifen und aur diese Weise direkt mit dem anderen Rahmenelement in Verbindung stehen.

Besonders vorteilhaft ist es, wenn das Fixierglied relativ zu beiden Rahmenelementen bewegbar ist. Auf diese Weise kann das mit dem einen Rahmenelement beweglich verbundene Fixierglied in die Ausnehmungen des anderen Rahmenelements eingreifen und stets mit dem einen Rahmenelement verbunden bleiben.

Gemäß einer vorteilhaften Ausgestaltung kann vorgesehen sein, daß das Fixierglied über ein kraftschlüssig mit diesem verbundenes Betätigungsglied bewegbar ist. Der Vorteil ist darin zu sehen, daß das Fixierglied versteckt angeordnet werden kann. Nur das Betätigungsglied muß einer Bedienperson der Kopfklemme zugänglich sein. Außerdem kann zum leichteren Betätigen des Fixierglieds ein entsprechender Hebel am Betätigungsglied vorgesehen sein. Darüber hinaus ist es denkbar, daß ein oder mehrere Fixierglieder durch ein einzelnes Betätigungsglied bewegbar sind. Auf diese Weise ergibt sich ein Schnellspannmechanismus für die Kopfklemme.

Bei einer anderen bevorzugten Ausführungsform der Erfindung kann vorgesehen sein, daß das Fixierglied in Richtung auf die Ausnehmungen hin verschiebbar an einem Rahmenelement gelagert ist. Die verschwenkte Kopfklemme kann also allein dadurch in ihrer Position fixiert werden, daß das Fixierglied in die Ausnehmung hineingeschoben wird. Dies ermöglicht eine besonders schnelle und einfache Handhabung der Kopfklemme.

Bei einer anderen Ausführungsform der Erfindung kann vorgesehen sein, daß das Fixierglied in Richtung auf die Ausnehmungen hin verschwenkbar an dem einen Rahmenelement gelagert ist. Eine verschwenkbare Lagerung des Fixierglieds ermöglicht es, sich aufgrund dieser Art der Lagerung ergebende Hebel an dem Fixierglied zu nutzen, was ein besonders kraftsparendes Verriegeln oder Lösen mit dem Fixierglied ermöglicht.

Besonders vorteilhaft ist es, wenn die Ausnehmungen durch die Zähne eines Zahnrades gebildet werden, dessen Umfang sich maximal über einen Winkelbereich von 360° erstreckt. Zahnräder sind maschinell sehr einfach und kostengünstig herzustellen. Außerdem kann es ausreichend sein, nur einen begrenzten Winkelbereich des Zahnrades mit Zähnen zu versehen, für den eine Fixierung der Kopfklemme in ihrer Position gewünscht wird. Dies reduziert zusätzlich den Herstellungsaufwand.

Vorteilhaft dabei ist es, wenn die Zähne mindestens einseitig unterschnitten sind. Dies hat den Vorteil, daß im Falle eines Eingreifens des Fixierglieds zwischen die Zähne des Zahnrades ein versehentliches Entfernen des Fixierglieds aus der Ausnehmung unmöglich wird, falls die Kopfklemme vorgespannt ist. Somit ergibt sich eine zusätzliche Sicherung gegen ein unbeabsichtigtes Lösen der Fixierstellung.

Gemäß einer bevorzugten Ausführungsform der Erfindung kann vorgesehen sein, daß nur jeweils eines der Fixierglieder in die Fixierstellung bringbar ist. Auf diese Weise kann bei entsprechender Anordnung der Fixierglieder relativ zu den Ausnehmungen eine feinere Unterteilung für mögliche Schwenkpositionen der Kopfklemme vorgegeben werden. D. h. einerseits, daß sich die Zahl der Fixierstellungen erhöht und andererseits, daß die Fixierglieder und die korrespondierenden Ausnehmungen so groß ausgebildet sein können, daß eine optimale Kraftübertragung ermöglicht wird. Dadurch wird zusätzlich das Spiel der Rahmenelemente relativ zueinander verringert und gleichzeitig die mechanische Stabilität der Kopfklemme erhöht.

Günstig ist es, wenn die Vorsprünge an den Fixiergliedern entsprechend der Anzahl der Fixierglieder jeweils um einen dieser Anzahl reziproken Bruchteil einer Zahnteilung des Zahnrades versetzt angeordnet sind. Auf diese Weise wird die Zahl der Zähne des Zahnrades virtuell um einen Faktor entsprechend der Anzahl der Fixierglieder vervielfacht. Das bedeutet beispielsweise, daß sich bei einer Zähnezahl von 20 auf einem Viertelskreisumfang und bei vier Fixiergliedern, eine mögliche Zahl der Fixierstellungen von 80 ergibt. Statt die Zahl der Zähne zu erhöhen und dadurch die Ausnehmungen zu verkleinern, können die Zähne größer ausgebildet werden, wodurch die Stabilität der Kopfklemme erhöht wird und größere Kräfte von der Schwenkpositionsfixierungsvorrichtung aufgenommen werden können.

Grundsätzlich kann vorgesehen sein, daß die Schwenkpositionsfixierungsvorrichtung mindestens ein elastisches Andrückelement aufweist, das das Fixierglied in der Fixierstellung hält. Auf diese Weise wird sichergestellt, daß die Rahmenelemente relativ zueinander stets fixiert sind. Es ist folglich nur dann notwendig, die Fixierglieder zu bewegen, wenn die Rahmenelemente relativ zueinander verschwenkt werden sollen. Dies bietet vor allem die Sicherheit, daß eine Fixierung der Rahmenelemente nicht vergessen wird. Eine Bedienperson, die die Kopfklemme in eine beliebige Position verschwenken möchte, muß zunächst alle Fixierglieder lösen, beispielsweise über das Betätigungsglied. Ansonsten werden die Fixierglieder durch das Andrückelement in den Ausnehmungen gehalten.

Vorteilhaft ist es dabei, wenn das elastische Andrückelement durch eine Blattfeder gebildet wird. Eine Blattfeder ist günstig herzustellen und einfach montierbar. Außerdem weist eine Blattfeder relativ große Anlageflächen auf. Auf diese Weise können auch mehrere Fixierglieder gleichzeitig von einer Blattfeder in der Fixierstellung gehalten werden.

Bei einer bevorzugten Ausführungsform der vorliegenden Erfindung kann vorgesehen sein, daß alle Fixierglieder gleichzeitig durch das Betätigungsglied in ihre Lösungsstellung bringbar sind. Dadurch wird eine schnelle Verstellung der Schwenkposition ermöglicht. Wird das Betätigungsglied nicht betätigt, dann verrasten die Fixierglieder in den Ausnehmungen, die Kopfklemme ist in einer definierten Schwenkposition fixiert. Erst durch Betätigung des Betätigungsglieds werden alle Fixierglieder gleichzeitig in ihre Lösungsstellung übergeführt. Jetzt können die Rahmenelemente der Kopfklemme relativ zueinander verschwenkt werden.

Grundsätzlich kann vorgesehen sein, daß ein Rahmenelement ein Rahmenteil und ein mit diesem verbundenes Stützteil aufweist, daß das Rahmenteil und das Stützteil um die gemeinsame Rotationsachse schwenkbar sind und einen Verschwenkungswinkel festlegen und daß das Rahmenteil relativ zum Stützteil bei jedem möglichen Verschwenkungswinkel fixierbar ist. Durch die oben beschriebene Fixierung einer Schwenkposition durch Fixierglieder und korrespondierende Ausnehmungen ist noch keine Feinjustage an der Kopfklemme möglich. Eine solche Feineinstellung wird erst durch die Trennung des Rahmenelements in das Rahmenteil und das Stützteil möglich. Im Gegensatz zu der Schwenkpositionsfixierungsvorrichtung ist zwischen dem Rahmenteil und dem Stützteil jeder mögliche Verschwenkungswinkel fixierbar. Somit können die Dorne in jede mögliche Position zum Festlegen eines Kopfes gebracht werden.

Dabei kann vorgesehen sein, daß das Rahmenteil relativ zum Stützteil über eine Verschwenkungswinkelverstellvorrichtung definiert verschwenkbar und fixierbar ist und daß die Verschwenkungswinkelverstellvorrichtung durch eine Schraubverbindung verstellbar ist. Durch die zusätzliche Verschraubung kann gleichzeitig das Rahmenteil relativ zum Stützteil verschwenkt und fixiert werden. Der Hauptvorteil dieser Ausgestaltung ist darin zu sehen, daß durch eine einzige Verschraubung eine Feinjustage der Kopfklemme möglich ist. Keine weiteren Verschraubungen sind darüber hinaus nötig.

Besonders vorteilhaft ist es, wenn die Fixierglieder an dem Stützteil angeordnet sind. Durch die Verbindung von dem Stützteil mit dem Rahmenteil kann das Rahmenteil relativ zu dem anderen Rahmenelement verschwenkt werden. Eine Fixierung dieser Schwenkposition wird über die Fixierglieder, die an dem Stützteil angeordnet sind, und die an dem anderen Rahmenelement angeordneten Ausnehmungen realisiert. Es wird also das Stützteil relativ zu dem anderen Rahmenelement in seiner Position fixiert. Ausgehend hiervon kann über die Schraubverbindung das Rahmenteil relativ zum Stützteil verschwenkt und fixiert werden. Die Grobeinstellung der Kopfklemme wird also über eine Bewegung des Stützteils relativ zum anderen Rahmenelement vorgenommen, die Feinjustage über eine Bewegung des Stützteils relativ zu dem damit verbundenen Rahmenteil.

Grundsätzlich kann vorgesehen sein, daß die Rahmenelemente Durchtrittsöffnungen für eine die Rotationsachse festlegende Lagerwelle aufweisen und mit dieser ein Gleitlager bilden. Die gleitende Anordnung der Rahmenelemente reduziert das Kippspiel quer zur Rotationsachse auf ein Minimum. Darüber hinaus wird die Rotationsachse durch die Symmetrieachse der Lagerwelle festgelegt.

Vorteilhaft dabei ist es, wenn die Rahmenelemente im Bereich der Lagerwelle quer zur Rotationsachse verlaufende Gleitflächen aufweisen, über die sie aneinander anliegen. Auf diese Weise können sich die Rahmenelemente im Bereich der Rotationsachse aneinander abstützen. Durch die spielfreie Gleitlagerung auf der Lagerwelle und die aneinander anliegenden Gleitflächen kann das Kippspiel zusätzlich verringert werden.

Besonders vorteilhaft ist es, wenn ein Rahmenelement fest mit der Lagerwelle verbunden ist. Auf diese Weise wird ein zusätzliches Spiel zwischen diesem Rahmenelement und der Lagerwelle von vornherein unmöglich, was die mechanische Stabilität der Kopfklemme erhöht.

Grundsätzlich kann vorgesehen sein, daß die Rahmenelemente relativ zueinander in Richtung der Rotationsachse fixiert sind. Die Rahmenelemente können also in Richtung der Rotationsachse nicht voneinander entfernt werden. Durch diese Fixierung kann ein benötigtes, wenn auch geringes Spiel zwischen den Rahmenelementen und der Lagerwelle eingestellt werden. Jedoch nur so viel, daß es zu keinem Verkippen der Rahmenelemente relativ zueinander kommen kann.

Gemäß einer bevorzugten Ausführungsform der Erfindung kann grundsätzlich vorgesehen sein, daß die Rahmenelemente im wesentlichen L-förmig sind. Diese Form der Rahmenelemente ist besonders günstig, um einen Kopf optimal zu umgeben. Außerdem bietet die L-Form im wesentlichen zwei gerade Schenkelabschnitte, an denen weitere Zusatzinstrumente befestigt werden können.

Vorteilhaft ist es, wenn das eine Rahmenelement an dem freien Ende einen Dorn aufweist, dessen Längsachse im wesentlichen auf das freie Ende des anderen Rahmenelements hin weist. Durch diese Ausrichtung der Dornachse ist eine optimale Kraftübertragung vom Rahmenelement über den Dorn auf den Kopf gegeben.

Günstig ist es, wenn das andere Rahmenelement an seinem freien Ende eine Wippenhalterung, deren Längsachse im wesentlichen auf das freie Ende des anderen Rahmenelements hin weist, wenn die Wippenhalterung einen quer zu ihrer Längsachse in seinem Mittelbereich schwenkbar gelagerten, im wesentlichen C-förmigen Bügel aufweist und wenn der Bügel an seinen freien Enden jeweils einen Dorn aufweist, der im wesentlichen auf das freie Ende des anderen Rahmenelements hin weist. Durch die Verlagerung der Grobeinstellung und der Feineinstellung an eine Basis der Kopfklemme ist es nicht mehr nötig, die einzelnen Dorne getrennt verstellbar zu machen. Durch die Wippenhalterung kann jeder beliebige Verschwenkungswinkel zwischen den Rahmenelementen dergestalt ausgeglichen werden, daß der Dorn am anderen Rahmenelement jeweils genau auf den schwenkbaren Mittelbereich des Bügels hin weist. Dabei wäre auch denkbar, daß die Wippenhalterung am Rahmenelement um ihre Längsachse rotierbar gelagert wird und in jeder möglichen Rotationsstellung durch eine Verriegelungsvorrichtung fixierbar ist. Außerdem ist es möglich, den einzelnen Dorn mit der Wippenhalterung zu vertauschen, je nachdem, wie groß der Platzbedarf für zusätzliche Instrumente ist, die an der Kopfklemme befestigt werden müssen.

Gemäß einer bevorzugten Ausführungsform der Erfindung kann vorgesehen sein, daß die Rahmenelemente im Querschnitt ein Profil aufweisen, das komplementär zu dem Profil einer entsprechenden Aufnahme einer Standardhalterung für weitere Instrumente geformt ist. Auf diese Weise wird die Kopfklemme universell einsetzbar für bereits vorhandene Halterungen zur Festlegung weiterer Instrumente.

Außerdem ist es günstig, wenn die Rahmenelemente im Bereich der Rotationsachse an ihrer jeweiligen Außenseite ein Profil aufweisen, das komplementär zu dem Profil einer Aufnahme einer standardisierten Kopfklemmenhaltevorrichtung geformt ist. Durch diese spezielle Profilierung kann die Kopfklemme auch in die bereits für herkömmliche Kopfklemmen verwendeten Kopfklemmenhaltevorrichtungen eingespannt werden. Dies ermöglicht einen Austausch der Kopfklemmen der eingangs beschriebenen Art durch die neu vorgeschlagenen Kopfklemmen unter Weiterbenutzung der vorhandenen Kopfklemmenhaltevorrichtungen.

Die nachfolgende Beschreibung einer bevorzugten Ausführungsform der Erfindung dient im Zusammenhang mit der Zeichnung der näheren Erläuterung. Es zeigen:
- Fig. 1: eine Seitenansicht einer Kopfklemme in einer Richtung quer zur Rotationsachse;
- Fig. 2: eine Seitenansicht einer Kopfklemme in Richtung der Rotationsachse;
- Fig. 3: eine Schnittansicht durch eine Kopfklemme entlang der Linie 3-3 aus Fig. 2; und
- Fig. 4: eine Querschnittsansicht entlang der Linie 4-4 aus Fig. 3.

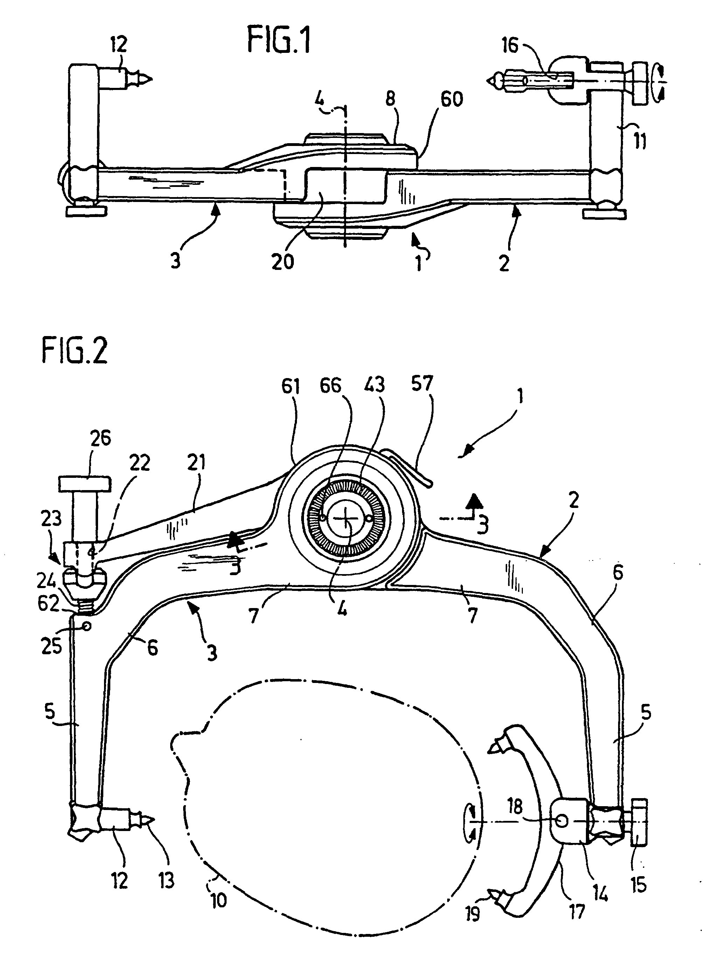

In den Figuren 1 und 2 ist eine insgesamt mit dem Bezugszeichen 1 versehene Kopfklemme dargestellt, die im wesentlichen durch zwei nahezu identische L-förmige Klemmbügel 2 und 3 gebildet wird. Die beiden Klemmbügel 2 und 3 sind relativ zueinander um eine Drehachse 4 verschwenkbar. Die durch drei geradlinige Bügelabschnitte gebildeten Klemmbügel 2 und 3 weisen einen im wesentlichen rechteckigen Querschnitt auf. Ausgehend von einem Endabschnitt 5 ist ein Mittelabschnitt 6 um etwa 45° abgewinkelt, der nach einer weiteren abgerundeten 45°-Abwinkelung in einen Basisabschnitt 7 übergeht. Auf diese Weise ergibt sich ein im wesentlichen L-förmiger Klemmbügel 2 bzw. 3. Die Länge der Abschnitte 5 bis 7 ist im wesentlichen identisch.

Am freien Ende des Basisabschnitts 7 schließt sich an diesen seitlich versetzt eine scheibenförmige Lageraufnahme 8 an, die eine zur Drehachse 4 rotationssymmetrische Lagerbohrung 9 aufweist, deren Durchmesser in etwa dem halben Durchmesser der Lageraufnahme 8 entspricht. Die Dicke der Lageraufnahme 8 in Richtung der Drehachse 4 entspricht etwa der Dicke des Basisabschnitts 7 in Richtung der Drehachse 4. Der Basisabschnitt 7 des Klemmbügels 2 bzw. 3 geht dabei mit seiner zusammen mit dem Endabschnitt 5 den 90°-Winkel bildenden Seite tangential in die äußere Umfangswandung 60 der Lageraufnahme 8 über.

Die beiden Klemmbügel 2 und 3 bilden so eine im wesentlichen C-förmige Kopfklemme 1, die zum Lagern eines Kopfes 10 denselben halbringförmig umgibt. Durch die unsymmetrische Anordnung der Lageraufnahmen 8 an den Basisabschnitten 7 ergibt sich eine halbkreisförmige Ausbuchtung 61 der Lageraufnahmen 8 auf der dem Kopf 10 abgewandten Seite der Kopfklemme 1.

An den freien Enden der Endabschnitte 5 sind parallel zur Drehachse 4 einseitig abstehende Lagerpfosten 11 angeordnet. Der mit dem Klemmbügel 3 verbundene Lagerpfosten 11 trägt dabei am Ende einer senkrecht vom Lagerpfosten 11 und dem Endabschnitt 5 abstehenden Dornhalterung 12 einen Einzeldorn 13, der die Verlängerung der rotationssymmetrischen Dornhalterung 12 bildet und auf den am Klemmbügel 2 angeordneten, gegenüberliegenden Lagerpfosten 11 hin weist.

Am Ende des Lagerpfostens 11 des Klemmbügels 2 ist eine Wippenhalterung 14 angeordnet, die in ähnlicher Weise wie die Dornhalterung 12 einseitig vom Lagerpfosten 11 absteht und auf die Dornhalterung 12 hin weist. Die Wippenhalterung 14 ist am Endabschnitt 5 des Klemmbügels 2 um ihre Längsachse rotierbar gelagert und kann mit einer Feststellschraube 15 fixiert werden, die auf der dem Kopf 10 abgewandten Seite der Kopfklemme 1 angeordnet ist. Auf ihrer dem Kopf 10 zugewandten Seite weist die Wippenhalterung 14 eine Aufnahmenut 16 auf, in der ein C-förmiger Wippenbügel 17 in einer Ebene schwenkbar gelagert ist, die parallel zu der durch die Klemmbügel 2 und 3 aufgespannten Ebene verläuft. Die um ihre Längsachse rotierbare und fixierbare Lagerung der Wippenhalterung 14 ermöglicht auch eine Verschwenkung des Wippenbügels 17 in einer Ebene, die nicht parallel zu der durch die Klemmbügel 2 und 3 aufgespannten Ebene verläuft. Die Schwenkachse des von der Wippenhalterung 14 weg gekrümmten Wippenbügels 17 wird durch die Symmetrieachse eines Lagerstifts 18 gebildet, wobei die Symmetrieachse des Lagerstifts 18 parallel zu einer Längsachse des Lagerpfostens 11 verläuft. Der an seinem mittleren Bereich gelagerte Wippenbügel 17 weist an seinen freien Enden jeweils einen Klemmdorn 19 auf, der quer zur Krümmung des Wippenbügels 17 von diesem absteht und in etwa auf das Zentrum des Kopfes 10 hin weist.

Durch die seitliche Versetzung der Lageraufnahmen 8 relativ zu den Basisabschnitten 7 wird ein im wesentlichen zylinderförmiger freier Bereich gebildet. In diesen hinein ragt formschlüssig an die Lageraufnahmen 8 anschließend ein in seiner Außenkontur zylindrisches Lageraufnahmeteil 20, das ähnlich der Lageraufnahme 8 tangential am Ende eines Stützträgers 21 anschließt. Das Lageraufnahmeteil 20 und der Stützträger 21 verlaufen in der gleichen Ebene wie die drei Abschnitte 5, 6 und 7. Der Stützträger 21 erstreckt sich im wesentlichen parallel zum Basisabschnitt 7 des Klemmbügels 3, ist jedoch so lang, daß er die gedachte Verlängerung des Endabschnitts 5 etwas überragt.

Dieses freie Ende des Stützträgers 21 weist eine Bohrung 22 auf, deren Symmetrieachse in Richtung der Längsachse des Endabschnitts 5 weist. In dieser Bohrung 22 ist ein nicht näher dargestellter Spindelantrieb 23 angeordnet, der über ein Verbindungsglied 24 mit dem Klemmbügel 3 verbunden ist. Das Verbindungsglied 24 greift am Rahmenelement 3 im Übergangsbereich zwischen dem Mittelabschnitt 6 und dem Endabschnitt 5 an einer Schulter 62 des Endabschnitts 5 an und ist dabei in der durch Klemmbügel 2 und 3 aufgespannten Ebene schwenkbar um einen weiteren Lagerstift 25 gelagert. Durch Drehen einer Verstellschraube 26 kann der Klemmbügel 3 relativ zum Stützträger 1 um die Drehachse 4 verschwenkt werden.

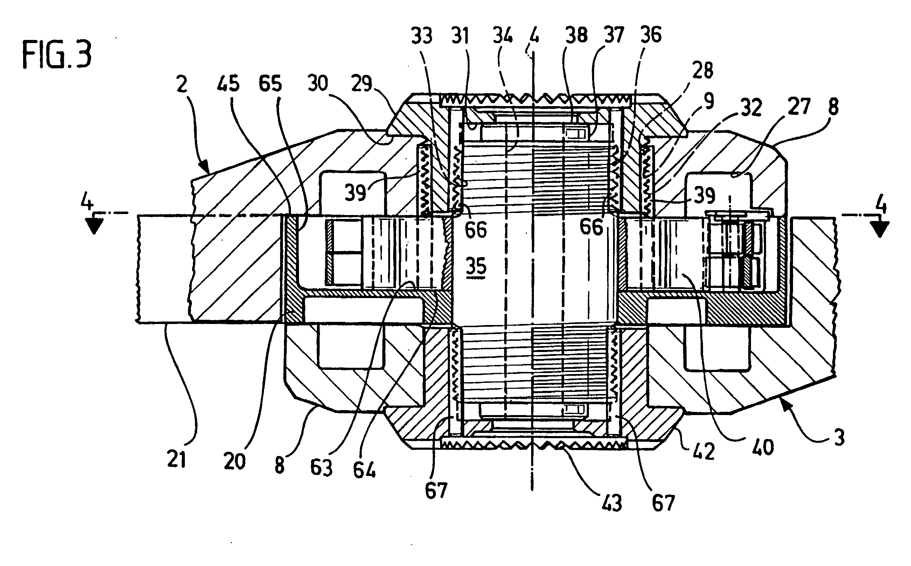

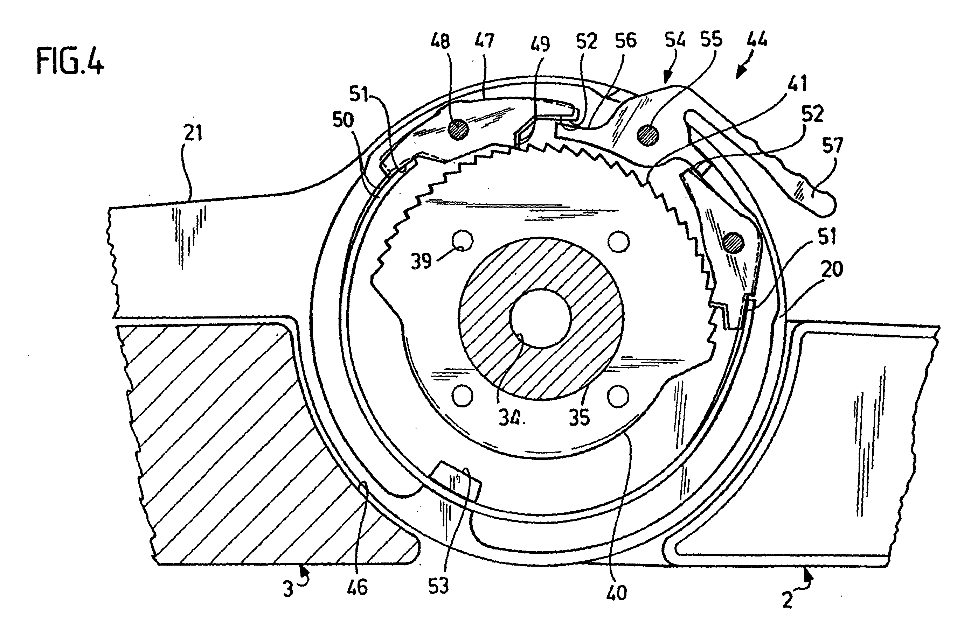

Bislang wurde lediglich die Geometrie der Kopfklemme 1 näher beschrieben. Im folgenden soll erläutert werden, wie die Klemmbügel 2 und 3 relativ zueinander verschwenkt werden können. In den Figuren 3 und 4 ist dazu die Verbindung der Klemmbügel 2 und 3 im Bereich der Drehachse 4 detailliert dargestellt.

Beide Lageraufnahmen 8 der Klemmbügel 2 bzw. 3 weisen eine Ringnut 27 auf, die jeweils zum Lageraufnahmeteil 20 hin offen ist. Die Lageraufnahme 8 des Klemmbügels 2 ist mit einem Innengewinde 28 versehen. In die mit dem Innengewinde 28 versehene Lagerbohrung 9 des Klemmbügels 2 ist eine Lagerhülse 29 eingeschraubt, von der auf der dem Lageraufnahmeteil 20 abgewandten Seite über den Durchmesser der Hülse hinaus ein Vorsprung 30 radial von der Drehachse 4 weg absteht, welcher als Anschlag der Lagerhülse 29 an der Lageraufnahme 8 dient. In der Verlängerung des Vorsprungs 30 erstreckt sich in Richtung auf die Drehachse 4 hin ein durch einen Vorsprung gebildeter Wellenanschlag 31. Die Lagerhülse 29 weist ferner ein zu dem Innengewinde 28 entsprechendes Außengewinde 32 auf. Zusätzlich ist die Lagerhülse 29 mit einem Innengewinde 33 versehen.

Eine zylindrische, mit einer zentralen Wellenbohrung 34 versehene Lagerwelle 35 ist an ihren beiden Enden mit einem Außengewinde 36 versehen, das sich jeweils über etwa ein Drittel der Länge der Lagerwelle 35 erstreckt. Über den Bereich der Außengewinde 36 hinaus nimmt der Durchmesser der Lagerwelle 35 einstufig ab. Dieser Wellenabschnitt 37 ist gewindefrei. Die Lagerwelle 35 ist mit ihrem einen Ende in das Innengewinde 33 der Lagerhülse 29 eingeschraubt. Sie schlägt dabei mit einer Stirnkante 38 des Wellenabschnitts 37 am Wellenanschlag 31 an.

Die Verschraubung der Lageraufnahme 8 mit der Lagerwelle 35 ist durch zwei Sicherungsstifte 66 zusätzlich gegen eine unbeabsichtigte Verdrehung gesichert. Die Sicherungsstifte 66 sind parallel zur Drehachse 4 von außen durch Bohrungen eingeführt, die einander, bezogen auf die Drehachse 4, diametral gegenüberliegen. Die Bohrungen für die Sicherungsstifte 66 sind so angeordnet, daß sie sowohl das Innengewinde 33 der Lagerhülse 29 als auch das Außengewinde 36 der Lagerwelle 35 erfassen. Auf diese Weise ist die Lagerwelle 35 drehfest mit der Lagerhülse 29 verbunden.

Zwischen ihren beiden mit dem Außengewinde 36 versehenen Enden ist die Lagerwelle 35 glatt. Ausgehend von der beschriebenen Verschraubung mit der Lagerhülse 29 wird der glatte Abschnitt der Lagerwelle 35 auf etwa zwei Drittel seiner Erstreckung in axialer Richtung von einem Zahnrad 40 umgeben, das etwa auf der Hälfte seines Umfangs mit einseitig unterschnittenen Zähnen 41 versehen ist. Das Zahnrad 40 ist mit vier Sicherungsstiften 39 drehfest mit der Lagerhülse 29 und der Lageraufnahme 8 verbunden. Die Sicherungsstifte 39 sind in Bohrungen eingesetzt, die relativ zur Drehachse 4 jeweils paarweise diametral gegenüber im Bereich des Außengewindes 32 der Lagerhülse 29 und des Innengewindes 28 der Lageraufnahme 8 angeordnet sind. Bei der Montage wird zunächst die Lagerhülse 29 in die Lageraufnahme 8 geschraubt und anschließend das Zahnrad 40 positioniert. Danach werden die drei Teile wie beschrieben mit vier Bohrungen versehen. Somit besteht eine drehfeste Verbindung des Zahnrads 40 zur Lagerhülse 29 und der Lageraufnahme 8 über die Sicherungsstifte 39 sowie ferner über die Sicherungsstifte 66 zur Lagerwelle 35.

Auf dem verbleibenden Drittel des gewindefreien Abschnitts der Lagerwelle 35 ist letztere von dem Lageraufnahmeteil 20 umgeben, das einen Innendurchmesser aufweist, der dem Außendurchmesser der Lagerwelle 35 entspricht. Dadurch ist das Lageraufnahmeteil 20 im wesentlichen spielfrei gegenüber der Lagerwelle 35 verdrehbar.

Auf der anderen Seite ist die Lagerwelle 35 analog wie oben für die Lagerhülse 29 beschrieben mit einer Gleitlagerhülse 42 verschraubt. Die Verschraubung ist analog über Sicherungsstifte 67 gesichert. Im Gegensatz zur Lagerhülse 29 weist die Gleitlagerhülse 42 jedoch kein Außengewinde auf. Ihr Außendurchmesser entspricht vielmehr dem Innendurchmesser der gewindefreien Lageraufnahme 8 des Klemmbügels 3, in die die Gleitlagerhülse 42 eingreift. Auf diese Weise ist der Klemmbügel 3 relativ zu der Gleitlagerhülse 42 drehbar gelagert. Die Gleitlagerhülse 42 ist gerade so weit in die Lageraufnahme 8 hineingeschraubt, daß kein Spiel in axialer Richtung zwischen dem Vorsprung 30, der Lageraufnahme 8 und dem Lageraufnahmeteil 20 verbleibt, jedoch eine gegenseitige Verschwenkung der beiden Klemmbügel 2 und 3 und des Stützträgers 21 noch möglich ist. Die Bohrungen für die Sicherungsstifte 67 werden erst dann abgebohrt, wenn die Gleitlagerhülse 42 auf die Lagerwelle 35 aufgeschraubt ist und die Verschwenkung der beiden Klemmbügel 2 und 3 und des Stützträgers 21 spielfrei eingestellt ist.

Die äußeren Stirnflächen der Lagerhülse 29 und der Gleitlagerhülse 42 sind mit verzahnten, kreisscheibenförmigen Profileinsätzen 43 versehen, deren Zähne nach außen in Richtung der Drehachse 4 weisen. Sie dienen als standardisiertes Aufnahmeelement zur Befestigung der Kopfklemme 1 an einer nicht dargestellten Grundeinheit, beispielsweise einer Halterung an einem Operationstisch.

Bislang wurde lediglich beschrieben, wie der Stützträger 21 relativ zum Klemmbügel 3 mittels des Spindelantriebs 23 verstellt werden kann. Relativ zu diesen beiden Teilen ist der Klemmbügel 2 völlig frei verschwenkbar. Um dessen Position relativ zum Stützträger 21 und dem Klemmbügel 3 zu fixieren, ist eine insgesamt mit dem Bezugszeichen 44 versehene Sperrvorrichtung vorgesehen, die im Detail in der Fig. 4 dargestellt ist.

Diese Sperrvorrichtung 44 wirkt direkt zwischen dem Lageraufnahmeteil 20 und dem Zahnrad 40. Im Bereich der Lagerwelle 35 liegen das Zahnrad 40 und das Lageraufnahmeteil 20 gleitend über Radialflächen 63 und 64 aneinander an. Im Außenbereich des Lageraufnahmeteils 20 erstreckt sich dieses als Begrenzungshülse 65 über den gesamten sich in axialer Richtung erstreckenden Bereich zwischen beiden Lageraufnahmen 8, wobei sich die Lageraufnahmen 8 an den Stirnflächen 45 des Lageraufnahmeteils 20 abstützen. Diese Stirnflächen 45 wirken gleichzeitig als Gleitflächen. Die auf das Lageraufnahmeteil 20 hin weisenden Basisabschnitte 7 der Klemmbügel 2 und 3 weisen eine Außenkontur 46 auf, die an den Außendurchmesser der Begrenzungshülse 65 des Lageraufnahmeteils 20 angepaßt ist. Um ein leichtes Verdrehen zu ermöglichen, ist der Außendurchmesser der Begrenzungshülse 65 des Lageraufnahmeteils 20 geringfügig kleiner als der Innendurchmesser der Außenkontur 46 des Basisabschnitts 7.

Das Lageraufnahmeteil 20 umgibt also mit seiner Begrenzungshülse 65 das Zahnrad 40 auf dessen gesamtem Umfang, wobei jedoch die Wandstärke der Begrenzungshülse 65 des Lageraufnahmeteils 20 so gering ist, daß etwa ein Abstand zum Zahnrad 40 verbleibt, der im Mittel etwa ein Viertel des Radius des Zahnrads 40 beträgt. In diesem freien Bereich sind vier Sperrklinken 47 verschwenkbar am Lageraufnahmeteil 20 gelagert. Für jeweils zwei Sperrklinken 47 ist ein gemeinsamer Sperrstift 48 vorgesehen, dessen Längsachse parallel zur Drehachse 4 verläuft. Die Sperrklinken 47 weisen eine im wesentlichen geschwungene und der inneren Umfangswandung der Begrenzungshülse zugewandte Außenkontur auf, die eine Verschwenkung innerhalb des freien Bereiches des Lageraufnahmeteils 20 ermöglicht. Die Sperrstifte 48 zur Lagerung der vier Sperrklinken 47 sind in Umfangsrichtung der Begrenzungshülse 65 jeweils um etwa 90° versetzt im freien Bereich des Lageraufnahmeteils 20 angeordnet. Jede der Sperrklinken 47 weist einen dem Zahnrad 40 zugewandten zahnförmigen Klinkenvorsprung 49 auf. Diese Klinkenvorsprünge 49 sind derart an den Sperrklinken 47 angeordnet, daß nur jeweils ein Klinkenvorsprung 49 vollständig zwischen zwei Zähne 41 des Zahnrads 40 eingreifen kann. Im Falle der vorliegenden vier Sperrklinken 47 sind die Klinkenvorsprünge 49 jeweils um ein Viertel des Zahnabstandes zweier Zähne 41 versetzt angeordnet. Alle vier Sperrklinken 47 werden gleichzeitig durch zwei Blattfedern 50 mit ihren Klinkenvorsprüngen 49 gegen das Zahnrad 40 gepreßt. Die Blattfeder 50 liegt dabei jeweils an einer Seitenfläche eines sich in Umfangsrichtung erstreckenden Andrückvorsprungs 51 der Sperrklinke 47 an. Die Blattfeder 50 selbst ist an einem Federbefestigungsvorsprung 53 festgelegt, der sich vom Umfang der Begrenzungshülse 65 des Lageraufnahmeteils 20 in Richtung auf die Drehachse 4 hin erstreckt. Dieser Federbefestigungsvorsprung 53 ist in etwa diametral zu den beiden Sperrstiften 48 auf der gegenüberliegenden Seite der Lagerwelle 35 angeordnet.

Die Sperrklinke 47 weist weiterhin einen Lösevorsprung 52 auf, der auf der anderen Seite des Sperrstifts 48 vom Andrückvorsprung 51 aus gesehen sich ebenfalls in Umfangsrichtung der Begrenzungshülse 65 erstreckend, angeordnet ist. Für alle Sperrklinken 47 ist eine gemeinsame Lösevorrichtung 54 vorgesehen. Die Lösevorrichtung 54 ist um einen Lösestift 55 verschwenkbar, der parallel zu den Sperrstiften 48 am Lageraufnahmeteil 20 angeordnet ist. Die zwischen den beiden Sperrklinken 47 angeordnete Lösevorrichtung 54 weist zwei sich in Umfangsrichtung erstreckende Ausklinkvorsprünge 56 auf, die an den Lösevorsprüngen 52 der Sperrklinken 47 anliegen. Ein mit der Lösevorrichtung 54 verbundener Lösehebel 57 steht aus dem Lageraufnahmeteil 20 hervor und im wesentlichen tangential in Umfangsrichtung der Begrenzungshülse 65 von diesem ab. Durch eine Bewegung des Lösehebels 57 in Richtung auf die Drehachse 4 hin ist die Lösevorrichtung 54 um den Lösestift 55 verschwenkbar, wodurch die Ausklinkvorsprünge 56 gegen die Lösevorsprünge 52 drücken, was wiederum zu einer Verschwenkung der Sperrklinken 47 führt. Beim Loslassen des Lösehebels 57 werden die Sperrklinken 47 durch die Kraft der Blattfedern 50 in die Fixierstellung zurück verschwenkt, und der Klinkenvorsprung 49 greift wieder zwischen zwei Zähne 41 des Zahnrads 40 ein.

Durch die Sperrvorrichtung 44 ist somit eine Grobverstellung der beiden Klemmbügel 2 und 3 relativ zueinander möglich. Zum Anlegen der Kopfklemme 1 wird zunächst der Lösehebel 57 der Lösevorrichtung 54 betätigt. Die beiden Klemmbügel 2 und 3 können nun beliebig verschwenkt werden. Die so geöffnete Kopfklemme 1 wird an den Kopf 10 herangeführt, und die Klemmbügel 2 und 3 werden so lange gegeneinander verschwenkt, bis der Einzeldorn 13 und die Klemmdorne 19 am Kopf 10 anliegen. Bei einer Verschwenkung zum Schließen der Kopfklemme 1 ist es nicht notwendig, den Lösehebel 57 gedrückt zu halten. Die Klinkenvorsprünge 49 gleiten nämlich an den nicht unterschnittenen Flanken der Zähne 41 des Zahnrads 40 auf, bis sie hinter die unterschnittenen Flanken des jeweils nächsten Zahns 41 des Zahnrads 40 rutschen. Können die Klemmbügel 2 und 3 nun nicht mehr weiter gegeneinander verschwenkt werden, dann wird durch Verdrehen der Verstellschraube 26 des Spindelantriebs 23 der Klemmbügel 3 relativ zum Stützträger 21 verschwenkt, und zwar so weit, bis der Kopf 10 unverrückbar zwischen den Einzeldorn 13 und die Klemmdorne 19 eingespannt ist. Ein unbeabsichtigtes Lösen dieser vorgespannten Position der Kopfklemme 1 ist unmöglich, denn einer der vier Klinkenvorsprünge 49 ist fest zwischen zwei Zähnen 41 hinter der unterschnittenen Flanke des einen Zahns 41 verkeilt.

Im Gegensatz zu herkömmlichen Kopfklemmen bietet die vorliegende Ausführungsform den Vorzug, daß zum Vorspannen des Stützträgers 21 relativ zum Klemmbügel 3 in Richtung des Verbindungsglieds 24 eine nicht dargestellte, große und damit sichere Druckfeder verwendet werden. Die Anordnung der mit der Verstellschraube 26 regulierbaren Druckfeder an einem Basisabschnitt 7 des Klemmbügels 3 hat ferner den Vorteil, daß das Federelement weder den Abdeckbereich der Operation, noch den Strahlengang für introoperative Röntgenuntersuchungen stört. Schlimmstenfalls kann die Druckfeder noch vom unsterilen Operationsbereich aus nachgezogen werden.

Die Dornhalterung 12 und die Wippenhalterung 14 sind frei austauschbar und gegebenenfalls auch am gegenüberliegenden Endabschnitt 5 befestigbar.

Für eine introoperative Röntgendiagnostik einschließlich der Computertomographie sind die Lagerpfosten 11 von beiden Seiten der Kopfklemme 1 her montierbar und austauschbar. Damit ist das Neurogranium, also das Gehirn bis zur Schädelbasis, im anterioren, posterioren und seitlichen Strahlengang weitgehend frei darstellbar. Bevorzugt werden die in die Bildebene hervorragenden Kopffixierungselemente, beispielsweise die Dornhalterung 12, der Einzeldorn 13, der Wippenbügel 17, die Wippenhalterung 14, die Klemmdorne 19, usw. aus röntgentolerantem Titan gefertigt.

Für die Fixierung von Zusatzgeräten weisen sämtliche seitlich zugänglichen Teile der Kopfklemme 1 ein identisches Aufnahmeprofil für Zangenelemente zur Fixierung von Halterungen für die Navigation, für Handauflagen usw. auf. Diese Teile der Kopfklemme 1, wie z. B. der Endabschnitt 5, der Mittelabschnitt 6 und der Basisabschnitt 7, sind entsprechend stärker dimensioniert.

## Patentansprüche

1. Kopfklemme (1) zum Lagern eines Kopfes (10) mit einem aus zwei Rahmenelementen (2, 3) gebildeten Rahmen, die jeweils erste Enden (7), die beweglich miteinander verbunden sind, und zweite Enden (5), die Dorne (13, 19) zur Fixierung des Kopfes (10) tragen, aufweisen, wobei die Rahmenelemente (2, 3) relativ zueinander um eine gemeinsame Rotationsachse (4) schwenkbar gelagert sind und der Rahmen (2, 3) eine Schwenkpositionsfixierungsvorrichtung mit mindestens einem Fixierglied (47) an einem der beiden Rahmenelemente umfaßt, **dadurch gekennzeichnet, daß** die Schwenkpositionsfixierungsvorrichtung (44) an dem Fixierglied (47) einen Vorsprung (49) aufweist und daß am anderen Rahmenelement (2) korrespondierende Ausnehmungen (41) angeordnet sind, in die der Vorsprung (49) in einer Fixierstellung eingreift und die Rahmenelemente (2, 3) relativ zueinander in ihrer Lage fixiert, und daß das Fixierglied (47) relativ zu den Ausnehmungen (41) von der Fixierstellung in eine Lösestellung bringbar ist, in der die Rahmenelemente (2, 3) relativ zueinander verschwenkbar sind.

2. Kopfklemme nach Anspruch 1, **dadurch gekennzeichnet, daß** die Ausnehmungen (41) drehfest mit dem anderen Rahmenelement (2) verbunden sind.

3. Kopfklemme nach einem der Ansprüche 1 oder 2, **dadurch gekennzeichnet, daß** das Fixierglied (47) relativ zu beiden Rahmenelementen (2, 3) bewegbar ist.

4. Kopfklemme nach Anspruch 3, **dadurch gekennzeichnet, daß** das Fixierglied (47) über ein kraftschlüssig mit diesem verbundenes Betätigungsglied (54) bewegbar ist.

5. Kopfklemme nach einem der Ansprüche 1 bis 4, **dadurch gekennzeichnet, daß** das Fixierglied (47) in Richtung auf die Ausnehmungen (41) hin verschiebbar an dem einen Rahmenelement (3) gelagert ist.

6. Kopfklemme nach einem der Ansprüche 1 bis 4, **dadurch gekennzeichnet, daß** das Fixierglied (47) in Richtung auf die Ausnehmungen (41) hin verschwenkbar an dem einen Rahmenelement (3) gelagert ist.

7. Kopfklemme nach einem der Ansprüche 1 bis 6, **dadurch gekennzeichnet, daß** die Ausnehmungen durch die Zähne (41) eines Zahnrades (40) gebildet werden, dessen Umfang sich maximal über einen Winkelbereich von 360° erstreckt.

8. Kopfklemme nach Anspruch 7, **dadurch gekennzeichnet, daß** die Zähne (41) mindestens einseitig unterschnitten sind.

9. Kopfklemme nach einem der Ansprüche 1 bis 8, **dadurch gekennzeichnet, daß** nur jeweils eines der Fixierglieder (47) in die Fixierstellung bringbar ist.

10. Kopfklemme nach einem der Ansprüche 7 bis 9, **dadurch gekennzeichnet, daß** die Vorsprünge (49) an den Fixiergliedern (47) entsprechend der Anzahl der Fixierglieder (47) jeweils um einen dieser Anzahl reziproken Bruchteil einer Zahnteilung des Zahnrades (40) versetzt angeordnet sind.

11. Kopfklemme nach einem der Ansprüche 1 bis 10, **dadurch gekennzeichnet, daß** die Schwenkpositionsfixierungsvorrichtung (44) mindestens ein elastisches Andrückelement (50) aufweist, das das Fixierglied (47) in der Fixierstellung hält.

12. Kopfklemme nach Anspruch 11, **dadurch gekennzeichnet, daß** das elastische Andrückelement durch eine Blattfeder (50) gebildet wird.

13. Kopfklemme nach einem der Ansprüche 4 bis 12, **dadurch gekennzeichnet, daß** alle Fixierglieder (47) gleichzeitig durch das Betätigungsglied (54) in ihre Lösestellung bringbar sind.

14. Kopfklemme nach einem der voranstehenden Ansprüche, **dadurch gekennzeichnet, daß** ein Rahmenelement ein Rahmenteil (3) und ein mit diesem verbundenes Stützteil (21) aufweist, daß das Rahmenteil (3) und das Stützteil (21) um die gemeinsame Rotationsachse (4) schwenkbar sind und einen Verschwenkungswinkel festlegen und daß das Rahmenteil (3) relativ zum Stützteil (21) bei jedem möglichen Verschwenkungswinkel fixierbar ist.

15. Kopfklemme nach Anspruch 14, **dadurch gekennzeichnet, daß** das Rahmenteil (3) relativ zum Stützteil (21) über eine Verschwenkungswinkelverstellvorrichtung (23, 24, 25; 26) definiert verschwenkbar und fixierbar ist und daß die Verschwenkungswinkelverstellvorrichtung (23, 24, 25, 26) durch eine Schraubverbindung verstellbar ist.

16. Kopfklemme nach einem der Ansprüche 14 oder 15, **dadurch gekennzeichnet, daß** die Fixierglieder (47) an dem Stützteil (21) angeordnet sind.

17. Kopfklemme nach einem der voranstehenden Ansprüche, **dadurch gekennzeichnet, daß** die Rahmenelemente (2, 3) Durchtrittsöffnungen (9) für eine die Rotationsachse (4) festlegende Lagerwelle (35) aufweisen und mit dieser ein Gleitlager bilden.

18. Kopfklemme nach Anspruch 17, **dadurch gekennzeichnet, daß** die Rahmenelemente (2, 3) im Bereich der Lagerwelle (35) quer zur Rotationsachse (4) verlaufende Gleitflächen (45) aufweisen, über die sie aneinander anliegen.

19. Kopfklemme nach einem der Ansprüche 17 oder 18, **dadurch gekennzeichnet, daß** ein Rahmenelement (2) fest mit der Lagerwelle (35) verbunden ist.

20. Kopfklemme nach einem der voranstehenden Ansprüche, **dadurch gekennzeichnet, daß** die Rahmenelemente (2, 3) relativ zueinander in Richtung der Rotationsachse (4) fixiert sind.

21. Kopfklemme nach einem der voranstehenden Ansprüche, **dadurch gekennzeichnet, daß** die Rahmenelemente (2, 3) im wesentlichen L-förmig sind.

22. Kopfklemme nach einem der voranstehenden Ansprüche, **dadurch gekennzeichnet, daß** das eine Rahmenelement (3) an dem freien Ende (5) einen Dorn (13) aufweist, dessen Längsachse im wesentlichen auf das freie Ende (5) des anderen Rahmenelements (2) hin weist.

23. Kopfklemme nach einem der voranstehenden Ansprüche, **dadurch gekennzeichnet, daß** das andere Rahmenelement (2) an seinem freien Ende (5) eine Wippenhalterung (14) aufweist, deren Längsachse im wesentlichen auf das freie Ende (5) des anderen Rahmenelements (3) hin weist, daß die Wippenhalterung (14) einen quer zu ihrer Längsachse in seinem Mittelbereich schwenkbar gelagerten im wesentlichen C-förmigen Bügel (17) aufweist und daß der Bügel (17) an seinen freien Enden jeweils einen Dorn (19) aufweist, der im wesentlichen auf das freie Ende (5) des anderen Rahmenelements (3) hin weist.

24. Kopfklemme nach einem der voranstehenden Ansprüche, **dadurch gekennzeichnet, daß** die Rahmenelemente (2, 3) im Querschnitt ein Profil aufweisen, das komplementär zu dem Profil einer entsprechenden Aufnahme einer Standardhalterung für weitere Instrumente geformt ist.

25. Kopfklemme nach einem der voranstehenden Ansprüche, **dadurch gekennzeichnet, daß** die Rahmenelemente (2, 3) im Bereich der Rotationsachse (4) an ihrer jeweiligen Außenseite ein Profil (43) aufweisen, das komplementär zu dem Profil einer Aufnahme einer standardisierten Kopfklemmenhaltevorrichtung geformt ist.

## Claims

1. A head clamp (1) for fixing a head (10), comprising a frame formed from two frame components (2, 3) each having first ends (7) movably connected to one another and second ends ( 5) carrying pins (13, 19) for fixing the head (10), wherein the frame components (2, 3) are mounted so as to be pivotable relative to one another about a common rotation axis (4), and the frame (2, 3) comprises a pivoted-position fixing device having at least one fixing member (47) on one of the two frame components, **characterised in that** the pivoted-position fixing device (44) has a projection (49) on the fixing member (47) and **in that** corresponding recesses (41), in which the projection (49) engages in a fixing position and fixes the frame components (2, 3) in their positions relative to one another, are arranged on the other frame component (2), and **in that** the fixing member (47) is movable in relation to the recesses (41) from the fixing position into a release position in which the frame components (2, 3) are pivotable relative to one another.

2. A head clamp according to claim 1, **characterised in that** the recesses (41) are connected to the other frame component (2) in a rotationally fixed manner.

3. A head clamp according to either one of claims 1 and 2, **characterised in that** the fixing member (47) is movable relative to both frame components (2, 3).

4. A head clamp according to claim 3, **characterised in that** the fixing member (47) is movable by an actuating member (54) mechanically connected thereto.

5. A head clamp according to any one of claims 1 to 4, **characterised in that** the fixing member (47) is mounted on the one frame component (3) so as to be displaceable towards the recesses (41).

6. A head clamp according to any one of claims 1 to 4, **characterised in that** the fixing member (47) is mounted on the one frame component (3) so as to be pivotable towards the recesses (41).

7. A head clamp according to any one of claims 1 to 6, **characterised in that** the recesses are formed by the teeth (41) of a toothed wheel (40), the circumference of which extends maximally over an angular range of 360°.

8. A head clamp according to claim 7, **characterised in that** the teeth (41) are undercut at least on one side.

9. A head clamp according to any one of claims 1 to 8, **characterised in that** only one of the fixing members (47) at a time is movable into the fixing position.

10. A head clamp according to any one of claims 7 to 9, **characterised in that** the projections (49) on the fixing members (47), corresponding to the number of fixing members (47), are each displaced by a fraction of a tooth pitch of the toothed wheel (40), the fraction being a reciprocal of the number of fixing members (47).

11. A head clamp according to any one of claims 1 to 10, **characterised in that** the pivoted-position fixing device (44) has at least one resilient pressure component (50) holding the fixing member (47) in the fixing position.

12. A head clamp according to claim 11, **characterised in that** the resilient pressure component is formed by a leaf spring (50).

13. A head clamp according to any one of claims 4 to 12, **characterised in that** all the fixing members (47) are simultaneously movable into their release position by the actuating member (54).

14. A head clamp according to any one of the preceding claims, **characterised in that** one frame component has a frame part (3) and a supporting part (21) connected thereto, **in that** the frame part (3) and the supporting part (21) are pivotable about the common rotation axis (4) and define a pivoting angle, and **in that** the frame part (3) is fixable relative to the supporting part (21) at any possible pivoting angle.

15. A head clamp according to claim 14, **characterised in that** the frame part (3) is pivotable and fixable relative to the supporting part (21) in a defined manner via a pivoting-angle adjusting device (23, 24, 25, 26) and **in that** the pivoting-angle adjusting device (23, 24, 25, 26) is adjustable by means of a screw connection.

16. A head clamp according to either one of claims 14 and 15, **characterised in that** the fixing members (47) are arranged on the supporting part (21).

17. A head clamp according to any one of the preceding claims, **characterised in that** the frame components (2, 3) have openings (9) for a bearing shaft (35) defining the rotation axis (4) and, together with the bearing shaft (35), form a sliding bearing.

18. A head clamp according to claim 17, **characterised in that** the frame components (2, 3) have sliding surfaces (45) which extend transversely to the rotation axis (4) in the region of the bearing shaft (35) and via which they rest against one another.

19. A head clamp according to either one of claims 17 and 18, **characterised in that** a frame component (2) is fixedly connected to the bearing shaft (35).

20. A head clamp according to any one of the preceding claims, **characterised in that** the frame components (2, 3) are fixed relative to one another in the direction of the rotation axis (4).

21. A head clamp according to any one of the preceding claims, **characterised in that** the frame components (2, 3) are substantially L-shaped.

22. A head clamp according to any one of the preceding claims, **characterised in that** the one frame component (3) has, at its free end (5), a pin (13), the longitudinal axis of which substantially extends towards the free end (5) of the other frame component (2).

23. A head clamp according to any one of the preceding claims, **characterised in that** the other frame component (2) has, at its free end (5), a rocker mounting (14), the longitudinal axis of which substantially extends towards the free end (5) of the other frame component (3), **in that** the rocker mounting (14) has a substantially C-shaped bow (17) pivotably mounted transversely to its longitudinal axis in its central region, and **in that** the bow (17) has, at each of its free ends, a pin (19) which substantially points towards the free end (5) of the other frame component (3).

24. A head clamp according to any one of the preceding claims, **characterised in that** the frame components (2, 3) have a cross-sectional profile complementary to the profile of a corresponding holder of a standard mounting for further instruments.

25. A head clamp according to any one of the preceding claims, **characterised in that** the frame components (2, 3) have, in the region of the rotation axis (4) on their respective outer surfaces, a profile (43) complementary to the profile of a holder of a standardised head-clamp mounting device.

## Revendications

1. Dispositif de serrage (1) pour une tête pour porter une tête (10), comportant un cadre, qui est formé par deux éléments de cadre (2, 3), qui comportent respectivement des premières extrémités (7), qui sont reliées entre elles de façon mobile, et des secondes extrémités (5), qui portent des mandrins (13, 19) servant à fixer la tête (10), les éléments de cadre (2, 3) étant montés de manière à pouvoir pivoter l'un par rapport à l'autre autour d'un axe de rotation commun (4), et le cadre (2, 3) comprenant un dispositif de fixation de la position pivotée comportant au moins un organe de fixation (47) sur l'un des deux éléments de cadre, **caractérisé en ce que** le dispositif (44) de fixation de la position pivotée comporte une partie saillante (49) sur l'organe de fixation (47), et que sur l'autre élément de cadre (2) sont formés des évidements correspondants (41), dans lesquels la partie saillante (49) s'engage, dans une position de fixation, et les éléments de cadre (2, 3) sont fixés réciproquement en position, et que l'organe de fixation (47) peut être amené par rapport aux évidements (41) depuis la position de fixation dans une position de dégagement, dans laquelle les éléments de cadre (2, 3) peuvent pivoter l'un par rapport à l'autre.

2. Dispositif de serrage pour une tête selon la revendication 1, **caractérisé en ce que** les évidements (41) sont reliés solidairement en rotation à l'autre élément de cadre (2).

3. Dispositif de serrage pour une tête selon l'une des revendications 1 ou 2, **caractérisé en ce que** l'organe de fixation (47) est déplaçable par rapport aux deux éléments de cadre (2, 3).

4. Dispositif de serrage pour une tête selon la revendication 3, **caractérisé en ce que** l'organe de fixation (47) est déplaçable par l'intermédiaire d'un organe d'actionnement (54) relié selon une liaison de force à cet organe de fixation.

5. Dispositif de serrage pour une tête selon l'une des revendications 1 à 4, **caractérisé en ce que** l'organe de fixation (47) est monté sur un élément de cadre (3) de manière à être déplaçable en direction des évidements (41).

6. Dispositif de serrage pour une tête selon l'une des revendications 1 à 4, **caractérisé en ce que** l'organe de fixation (47) est monté sur un élément de cadre (3) de manière à pouvoir pivoter en direction des évidements (41).

7. Dispositif de serrage pour une tête selon l'une des revendications 1 à 6, **caractérisé en ce que** les évidements sont formés par les dents (41) d'un pignon (40), dont la périphérie s'étend au maximum sur une plage angulaire de 360°.

8. Dispositif de serrage pour une tête selon la revendication 7, **caractérisé en ce que** les dents (41) sont chanfreinées au moins d'un côté au niveau de leur partie inférieure.

9. Dispositif de serrage pour une tête selon l'une des revendications 1 à 8, **caractérisé en ce que** seul respectivement l'un des organes de fixation (47) peut être amené dans la position de fixation.

10. Dispositif de serrage pour une tête selon l'une des revendications 7 à 9, **caractérisé en ce que** les parties saillantes (49) sont disposées sur les organes de fixation (47) en étant décalées, en fonction du nombre des organes de fixation (47), respectivement d'une fraction, inverse de ce nombre, d'un pas de répartition des dents du pignon (40).

11. Dispositif de serrage pour une tête selon l'une des revendications 1 à 10, **caractérisé en ce que** le dispositif (44) de fixation de la position pivotée comporte au moins un élément de serrage élastique (50), qui maintient l'organe de fixation (47) dans la position de fixation.

12. Dispositif de serrage pour une tête selon la revendication 11, **caractérisé en ce que** l'élément de serrage élastique est formé par un ressort à lame (50).

13. Dispositif de serrage pour une tête selon l'une des revendications 4 à 12, **caractérisé en ce que** tous les organes de fixation (47) peuvent être amenés simultanément par l'organe d'actionnement (54) dans leur position de libération.

14. Dispositif de serrage pour une tête selon l'une des revendications précédentes, **caractérisé en ce qu'**un élément de cadre comporte une partie de cadre (3) et une partie d'appui (21) reliée à cette partie de cadre, que la partie de cadre (3) et la partie d'appui (21) peuvent pivoter autour de l'axe de rotation commun (4) et fixent un angle de pivotement, et que la partie de cadre (3) peut être fixée par rapport à la partie d'appui (21) pour chaque angle de pivotement possible.

15. Dispositif de serrage pour une tête selon la revendication 14, **caractérisé en ce que** la partie de cadre (3) peut pivoter et peut être fixée de façon définie par rapport à la partie d'appui 21 par l'intermédiaire d'un dispositif (23, 24, 25, 26) de réglage de l'angle de pivotement et que le dispositif (23, 24, 25, 26) de réglage de l'angle de pivotement est réglable à l'aide d'une liaison vissée.

16. Dispositif de serrage pour une tête selon l'une des revendications 14 ou 15, **caractérisé en ce que** les organes de fixation (47) sont disposés sur la partie d'appui 21.

17. Dispositif de serrage pour une tête selon l'une des revendications précédentes, **caractérisé en ce que** les éléments de cadre (2, 3) comportent des ouvertures de passage (9) pour un arbre de support (35), qui fixe l'axe de rotation (4), et forment avec ce dernier un palier lisse.

18. Dispositif de serrage pour une tête selon la revendication 17, **caractérisé en ce que** les éléments de cadre (2, 3) comportent dans la zone de l'arbre de support (50), des surfaces de glissement (45), qui s'étendent transversalement par rapport à l'axe de rotation (4) et à l'aide desquelles ils s'appliquent l'un contre l'autre.

19. Dispositif de serrage pour une tête selon l'une des revendications 17 ou 18, **caractérisé en ce qu'**un élément de cadre (2) est relié de façon fixe à l'arbre de support (35).

20. Dispositif de serrage pour une tête selon l'une des revendications précédentes, **caractérisé en ce que** les éléments de cadre (2, 3) sont fixés l'un par rapport à l'autre dans la direction de l'axe de rotation (4).

21. Dispositif de serrage pour une tête selon l'une des revendications précédentes, **caractérisé en ce que** les éléments de cadre (2, 3) sont réalisés essentiellement en forme de L.

22. Dispositif de serrage pour une tête selon l'une des revendications précédentes, **caractérisé en ce qu'**un élément de cadre (3) comporte, sur l'extrémité libre (35) un mandrin (13), dont l'axe longitudinal est dirigé essentiellement vers l'extrémité libre (5) de l'autre élément de cadre (2).

23. Dispositif de serrage pour une tête selon l'une des revendications précédentes, **caractérisé en ce que** l'autre élément de cadre (2) comporte, sur son extrémité libre (5), un dispositif (14) de retenue à bascule, dont l'axe longitudinal est dirigé essentiellement vers l'extrémité libre (5) de l'autre élément de cadre (3), que le dispositif de retenue à bascule (14) possède un étrier essentiellement en forme de C (17), qui est monté de manière à pouvoir basculer transversalement par rapport à son axe longitudinal, dans sa partie médiane, et que l'étrier (17) comporte sur son extrémité libre respectivement un mandrin (19) qui est dirigé essentiellement sur l'extrémité libre (5) de l'autre élément de cadre (3).

24. Dispositif de serrage pour une tête selon l'une des revendications précédentes, **caractérisé en ce que** les éléments de cadre (2, 3) possèdent en coupe transversale un profil, qui est complémentaire du profil d'un logement correspondant d'un dispositif de support standard pour d'autres instruments.

25. Dispositif de serrage pour une tête selon l'une des revendications précédentes, **caractérisé en ce que** les éléments de cadre (2, 3) possèdent, dans la zone de l'axe de rotation (4), sur leur côté extérieur respectif un profil (43), qui est conformé de manière à être complémentaire du profil d'un logement d'un dispositif normalisé de retenue du dispositif de serrage pour une tête.
